# EUROPEAN PATENT APPLICATION

(11) **EP 4 349 830 A1**
(43) Date of publication of application: **10.04.2024**
(21) Application number: 22816207.9
(22) Date of filing: 02.06.2022
(51) Int. Cl.: C07D 403/04, A01N 43/52, A61K 31/4184, A61P 33/00, A61P 33/10

(54) **BENZIMIDAZOLE COMPOUND OR SALT THEREOF, CANINE FILARIASIS CONTROL AGENT CONTAINING SAME, AND METHOD OF USE THEREOF**

(30) Priority: 02.06.2021 JP 2021092798; 15.02.2022 JP 2022021150
(71) Applicant: Nihon Nohyaku Co., Ltd., Tokyo 104-8386 (JP)
(72) Inventor: FUKATSU, Kosuke, Kawachinagano-shi, Osaka 586-0094 (JP); TANAKA, Ryosuke, Kawachinagano-shi, Osaka 586-0094 (JP); FUJIHARA, Hirokazu, Kawachinagano-shi, Osaka 586-0094 (JP)
(74) Representative: Grünecker Patent- und Rechtsanwälte PartG mbB
(86) International application number: PCT/JP2022/022538
(87) International publication number: WO 2022/255462

(57) **Abstract**

An object of the present application is to provide a canine filariasis control agent for an animal, which is administered to a subject animal to exert an excellent effect and a method of using a canine filariasis control agent for a subject animal using the same. The present invention provides a benzimidazole compound represented by general formula (1): 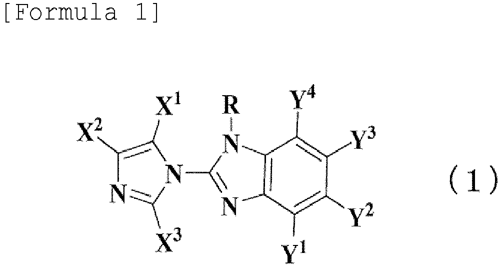 wherein R represents a hydrogen atom, or the like; X¹, X³, Y¹, Y², Y³, and Y⁴ represent a hydrogen atom or the like; and X² represents a substituted phenylalkyl group or the like, or a salt thereof. The present invention also provides a canine filariasis control agent for an animal, containing the same as an active ingredient, a method of using the same, and the like.

## Description

### Technical Field

The present invention relates to a benzimidazole compound or salt thereof, a canine filariasis control agent for an animal containing said compound as an active ingredient, and a method of use thereof.

### Background Art

Canine filariasis is caused by mosquito-borne canine filaria and occur in many animal species and pet species. The mechanism is as follows. When a mosquito ingests microfilariae (neonatal larval stage), the microfilariae repeatedly molt within body of the mosquito to develop into infected larvae (L3). When the mosquito bites an animal, the infected larvae reach the host skin to enter inside the animal and begin to grow. The infected larvae molt within 3 to 12 days to enter the fourth stage (L4) and stay in the subcutaneous tissue, abdomen, and thorax for about 2 months. The L4 larvae then undergo a final molt to become young adults and the adults reach the host heart and pulmonary arteries approximately 70 to 120 days after initial infection.

Meanwhile, it has been reported that a benzimidazole compound having a pyridyl group at position 2 is effective in controlling ectoparasites or endoparasites in animals (see, e.g., Patent Literature 1). The literature, however, has no disclosures of descriptions about benzimidazole compounds that have an imidazole group at position 2, and about insecticidal effects of the compounds on canine filariae.

### Citation List

### Patent Literature

Patent Literature 1: WO 2019/225663

### Summary of Invention

### Technical Problem

Melarsomine dihydrochloride has been known as a conventional canine filariasis control agent, and the drug is effective against both mature (adult) and immature canine filariae. Canine filariasis can also be prevented by macrolide preventives, and year-round prophylaxis is recommended, regardless of the animal's husbandry conditions. There is concern that long-term use of these drugs in animals may cause the development of resistance to the existing drugs. Therefore, there is a need for new drugs that are active against canine filariasis and can be used to treat infections caused by the filariae.

### Solution to Problem

The present inventors have intensively studied to solve the above problems, and as a result, have found that a benzimidazole compound represented by general formula (1) in which the compound has an imidazole group at position 2, or a salt thereof, not only has an excellent control effect on canine filariasis but also can solve the above problems, and have completed the present invention.

That is, the present invention relates to the following.
[1] A benzimidazole compound represented by general formula (1) 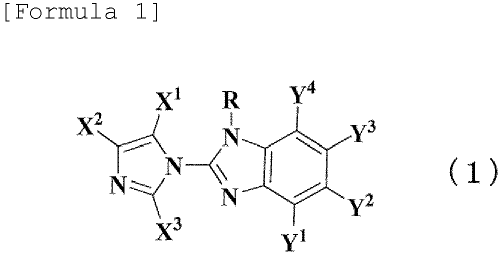 wherein
   R represents
      (a1) a hydrogen atom;
      (a2) a (C₁-C₆) alkyl group;
      (a3) a halo (C₁-C₆) alkyl group;
      (a4) a (C₂-C₆) alkenyl group;
      (a5) a (C₂-C₆)alkynyl group;
      (a6) a (C₁-C₆)alkoxy(C₁-C₆)alkyl group;
      (a7) a (C₁-C₆)alkylthio(C₁-C₆)alkyl group;
      (a8) a (C₁-C₆)alkoxycarbonyl(C₁-C₆)alkyl group;
      (a9) a (C₁-C₆)alkoxycarbonyl group;
      (a10) an aryl (C₁-C₆)alkyl group;
      (a11) an aryl(C₁-C₆)alkyl group having 1 to 5 identical or different substituents selected from (a) a halogen atom, (b) a (C₁-C₆)alkyl group, (c) a halo (C₁-C₆)alkyl group, (d) a (C₁-C₆)alkoxy group, (e) a halo (C₁-C₆)alkoxy group, (f) a (C₁-C₆)alkylthio group, (g) a halo (C₁-C₆)alkylthio group, (h) a (C₁-C₆)alkylsulfinyl group, (i) a halo (C₁-C₆)alkylsulfinyl group, (j) a (C₁-C₆)alkylsulfonyl group, (k) a halo (C₁-C₆)alkylsulfonyl group, (l) a nitro group, and (m) a trimethylsilyl group; (a12) an aryl (C₁-C₆)alkoxy(C₁-C₆)alkyl group;
      (a13) an aryl (C₁-C₆)alkoxy(C₁-C₆)alkyl group having 1 to 5 identical or different substituents selected from (a) a halogen atom, (b) a (C₁-C₆)alkyl group, (c) a halo (C₁-C₆)alkyl group, (d) a (C₁-C₆) alkoxy group, (e) a halo (C₁-C₆)alkoxy group, (f) a (C₁-C₆)alkylthio group, (g) a halo (C₁-C₆)alkylthio group, (h) a (C₁-C₆)alkylsulfinyl group, (i) a halo (C₁-C₆)alkylsulfinyl group, (j) a (C₁-C₆)alkylsulfonyl group, (k) a halo (C₁-C₆)alkylsulfonyl group, (l) a nitro group, and (m) a trimethylsilyl group; (a14) an R³R⁴N group, wherein R³ and R⁴ are identical or different, and represent (aa) a hydrogen atom, (ab) a (C₁-C₆)alkyl group, (ac) a (C₁-C₆)alkoxy group, (ad) a (C₁-C₆)alkoxycarbonyl group, (ae) a (C₁-C₆)alkylsulfonyl group, (af) a halo (C₁-C₆) alkylsulfonyl group, or (ag) a phenyl group;
      (a15) an R³R⁴N carbonyl group, wherein R³ and R⁴ are the same as above;
      (a16) an R³R⁴N thiocarbonyl group, wherein R³ and R⁴ are the same as above; or
      (a17) an R³R⁴N sulfonyl group, wherein R³ and R⁴ are the same as above;
   X¹ and X³ are identical or different, and represent
      (b1) a hydrogen atom;
      (b2) a halogen atom; or
      (b3) a (C₁-C₆)alkyl group;
   X² represents
      (c1) a (C₁-C₈)alkyl group;
      (c2) a (C₂-C₈)alkenyl group;
      (c3) a (C₂-C₈)alkynyl group;
      (c4) an aryl group;
      (c5) an aryl group having 1 to 5 identical or different substituents selected from (a) a halogen atom, (b) a (C₁-C₆)alkyl group, (c) a halo (C₁-C₆) alkyl group, (d) a (C₁-C₆)alkoxy group, (e) a halo (C₁-C₆) alkoxy group, (f) a (C₁-C₆)alkylthio group, (g) a halo (C₁-C₆) alkylthio group, (h) a (C₁-C₆)alkylsulfinyl group, (i) a halo (C₁-C₆)alkylsulfinyl group, (l) a nitro group, and (m) a trimethylsilyl group;
      (c6) an aryl (C₁-C₆) alkyl group; or
      (c7) an aryl(C₁-C₆) alkyl group having 1 to 5 identical or different substituents selected from (a) a halogen atom, (b) a (C₁-C₆)alkyl group, (c) a halo (C₁-C₆)alkyl group, (d) a (C₁-C₆)alkoxy group, (e) a halo (C₁-C₆)alkoxy group, (f) a (C₁-C₆)alkylthio group, (g) a halo (C₁-C₆)alkylthio group, (h) a (C₁-C₆)alkylsulfinyl group, (i) a halo (C₁-C₆)alkylsulfinyl group, (j) a (C₁-C₆)alkylsulfonyl group, (k) a halo (C₁-C₆)alkylsulfonyl group, (l) a nitro group, and (m) a trimethylsilyl group;
   Y¹ and Y⁴ are identical or different, and represent
      (d1) a hydrogen atom;
      (d2) a halogen atom; or
      (d3) a (C₁-C₆)alkyl group; and
   Y² and Y³ are identical or different, and represent
      (e1) a hydrogen atom;
      (e2) a halogen atom;
      (e3) a (C₁-C₆)alkyl group;
      (e4) a (C₁-C₆)alkoxy group;
      (e5) a (C₁-C₆)alkylthio group;
      (e6) a halo (C₁-C₆)alkyl group;
      (e7) a halo (C₁-C₆)alkoxy group;
      (e8) an aryloxy group;
      (e9) an aryloxy group having 1 to 5 identical or different substituents selected from (a) a halogen atom, (b) a (C₁-C₆) alkyl group, (c) a halo (C₁-C₆) alkyl group, (d) a (C₁-C₆) alkoxy group, (e) a halo (C₁-C₆) alkoxy group, (f) a (C₁-C₆) alkylthio group, (g) a halo (C₁-C₆) alkylthio group, (h) a (C₁-C₆) alkylsulfinyl group, (i) a halo (C₁-C₆)alkylsulfinyl group, (j) a (C₁-C₆) alkylsulfonyl group, (k) a halo (C₁-C₆) alkylsulfonyl group, (l) a nitro group, and (m) a trimethylsilyl group;
      (e10) an arylthio group;
      (e11) an arylthio group having 1 to 5 identical or different substituents selected from (a) a halogen atom, (b) a (C₁-C₆) alkyl group, (c) a halo (C₁-C₆) alkyl group, (d) a (C₁-C₆) alkoxy group, (e) a halo (C₁-C₆) alkoxy group, (f) a (C₁-C₆) alkylthio group, (g) a halo (C₁-C₆) alkylthio group, (h) a (C₁-C₆) alkylsulfinyl group, (i) a halo (C₁-C₆)alkylsulfinyl group, (j) a (C₁-C₆) alkylsulfonyl group, (k) a halo (C₁-C₆) alkylsulfonyl group, (l) a nitro group, and (m) a trimethylsilyl group;
      (e12) an arylcarbonyl group; or
      (e13) an arylcarbonyl group having 1 to 5 identical or different substituents selected from (a) a halogen atom, (b) a (C₁-C₆) alkyl group, (c) a halo (C₁-C₆) alkyl group, (d) a (C₁-C₆) alkoxy group, (e) a halo (C₁-C₆) alkoxy group, (f) a (C₁-C₆)alkylthio group, (g) a halo (C₁-C₆)alkylthio group, (h) a (C₁-C₆)alkylsulfinyl group, (i) a halo (C₁-C₆)alkylsulfinyl group, (j) a (C₁-C₆) alkylsulfonyl group, (k) a halo (C₁-C₆) alkylsulfonyl group, (l) a nitro group, and (m) a trimethylsilyl group,
   or a salt thereof.
[2] The benzimidazole compound according to [1] or a salt thereof, wherein R represents
   (a1) a hydrogen atom;
   (a2) a (C₁-C₆) alkyl group;
   (a3) a halo (C₁-C₆) alkyl group;
   (a4) a (C₂-C₆) alkenyl group;
   (a5) a (C₂-C₆)alkynyl group;
   (a6) a (C₁-C₆)alkoxy(C₁-C₆)alkyl group;
   (a7) a (C₁-C₆) alkylthio (C₁-C₆) alkyl group;
   (a8) a (C₁-C₆) alkoxycarbonyl (C₁-C₆) alkyl group;
   (a9) a (C₁-C₆)alkoxycarbonyl group;
   (a10) an aryl(C₁-C₆)alkyl group;
   (a12) an aryl(C₁-C₆)alkoxy(C₁-C₆)alkyl group;
   (a15) an R³R⁴N carbonyl group, wherein R³ and R⁴ are the same as above;
   (a16) an R³R⁴N thiocarbonyl group, wherein R³ and R⁴ are the same as above; or
   (a17) an R³R⁴N sulfonyl group, wherein R³ and R⁴ are the same as above;
   X¹ and X³ are both represent (b1) a hydrogen atom;
   X² represents
      (c1) a (C₁-C₈)alkyl group;
      (c3) a (C₂-C₈)alkynyl group;
      (c5) an aryl group having 1 to 5 identical or different substituents selected from (a) a halogen atom, (b) a (C₁-C₆)alkyl group, (c) a halo (C₁-C₆) alkyl group, (d) a (C₁-C₆)alkoxy group, (e) a halo (C₁-C₆) alkoxy group, (f) a (C₁-C₆)alkylthio group, (g) a halo (C₁-C₆) alkylthio group, (h) a (C₁-C₆)alkylsulfinyl group, (i) a halo (C₁-C₆)alkylsulfinyl group, (l) a nitro group, and (m) a trimethylsilyl group; or
      (c7) an aryl(C₁-C₆) alkyl group having 1 to 5 identical or different substituents selected from (a) a halogen atom, (b) a (C₁-C₆)alkyl group, (c) a halo(C₁-C₆)alkyl group, (d) a (C₁-C₆)alkoxy group, (e) a halo(C₁-C₆)alkoxy group, (f) a (C₁-C₆)alkylthio group, (g) a halo(C₁-C₆)alkylthio group, (h) a (C₁-C₆)alkylsulfinyl group, (i) a halo(C₁-C₆)alkylsulfinyl group, (j) a (C₁-C₆)alkylsulfonyl group, (k) a halo(C₁-C₆)alkylsulfonyl group, (l) a nitro group, and (m) a trimethylsilyl group;
   Y¹ and Y⁴ are identical or different, and represent
      (d1) a hydrogen atom;
      (d2) a halogen atom; or
      (d3) a (C₁-C₆)alkyl group;
   Y² and Y³ are identical or different, and represent
      (e1) a hydrogen atom;
      (e2) a halogen atom;
      (e3) a (C₁-C₆)alkyl group;
      (e5) a (C₁-C₆)alkylthio group;
      (e6) a halo(C₁-C₆)alkyl group;
      (e7) a halo(C₁-C₆)alkoxy group;
      (e8) an aryloxy group;
      (e10) an arylthio group; or
      (e12) an arylcarbonyl group.
[3] A canine filariasis control agent for an animal, containing the benzimidazole compound or a salt thereof according to any one of [1] to [2] as an active ingredient.
[4] A method of using a canine filariasis control agent for an animal, including administering an effective amount of the benzimidazole compound or a salt thereof according to any one of [1] to [2] to the animal.

### Advantageous Effect of Invention

The benzimidazole compound in which the compound has an imidazole group at position 2, or a salt thereof of the present invention, has excellent effects as a canine filariasis control agent.

### Description of Embodiments

In the definition of formula (1) of the benzimidazole compound in which the compound has an imidazole group at position 2, or a salt thereof of the present invention, "halo" means a "halogen atom" and represents a fluorine atom, a chlorine atom, a bromine atom, or an iodine atom.

The "(C₁-C₆)alkyl group" refers to a linear or branched alkyl group having 1 to 6 carbon atoms, such as a methyl group, an ethyl group, a normal propyl group, an isopropyl group, a normal butyl group, an isobutyl group, a secondary butyl group, a tertiary butyl group, a normal pentyl group, an isopentyl group, a tertiary pentyl group, a neopentyl group, a 2,3-dimethylpropyl group, a 1-ethylpropyl group, a 1-methylbutyl group, a 2-methylbutyl group, a normal hexyl group, an isohexyl group, a 2-hexyl group, a 3-hexyl group, a 2-methylpentyl group, a 3-methylpentyl group, a 1,1,2-trimethylpropyl group, or a 3,3-dimethylbutyl group. The " (C₂-C₆)alkenyl group" refers to a linear or branched alkenyl group having 2 to 6 carbon atoms, such as a vinyl group, an allyl group, an isopropenyl group, a 1-butenyl group, a 2-butenyl group, a 2-methyl-2-propenyl group, a 1-methyl-2-propenyl group, a 2-methyl-1-propenyl group, a pentenyl group, a 1-hexenyl group, or a 3,3-dimethyl-1-butenyl group. The " (C₂-C₆) alkynyl group" refers to a linear or branched alkynyl group having 2 to 6 carbon atoms, such as an ethynyl group, a 1-propynyl group, a 2-propynyl group, a 1-butynyl group, a 2-butynyl group, a 3-butynyl group, a 3-methyl-1-propynyl group, a 2-methyl-3-propynyl group, a pentynyl group, or a 1-hexynyl group.

The " (C₁-C₆) alkoxy group" refers to a linear or branched alkoxy group having 1 to 6 carbon atoms, such as a methoxy group, an ethoxy group, a normal propoxy group, an isopropoxy group, a normal butoxy group, a secondary butoxy group, a tertiary butoxy group, a normal pentyloxy group, an isopentyloxy group, a tertiary pentyloxy group, a neopentyloxy group, a 2,3-dimethylpropyloxy group, a 1-ethylpropyloxy group, a 1-methylbutyloxy group, and a normal hexyloxy group.

The " (C₁-C₆)alkylthio group" refers to a linear or branched alkylthio group having 1 to 6 carbon atoms, such as a methylthio group, an ethylthio group, a normal propylthio group, an isopropylthio group, a normal butylthio group, a secondary butylthio group, a tertiary butylthio group, a normal pentylthio group, an isopentylthio group, a tertiary pentylthio group, a neopentylthio group, a 2,3-dimethylpropylthio group, a 1-ethylpropylthio group, a 1-methylbutylthio group, a normal hexylthio group, an isohexylthio group, or a 1,1,2-trimethyl propylthio group. The "(C₁-C₆)alkylsulfinyl group" refers to a linear or branched alkylsulfinyl group having 1 to 6 carbon atoms, such as a methylsulfinyl group, an ethylsulfinyl group, a normal propylsulfinyl group, an isopropylsulfinyl group, a normal butylsulfinyl group, a secondary butylsulfinyl group, a tertiary butylsulfinyl group, a normal pentylsulfinyl group, an isopentylsulfinyl group, a tertiary pentylsulfinyl group, a neopentylsulfinyl group, a 2,3-dimethylpropylsulfinyl group, a 1-ethylpropylsulfinyl group, a 1-methylbutylsulfinyl groups, a normal hexysulfinyl group, an isohexysulfinyl group, or a 1,1,2-trimethylpropylsulfinyl group. The " (C₁-C₆)alkylsulfonyl group" refers to a linear or branched alkylsulfonyl group having 1 to 6 carbon atoms, such as a methylsulfonyl group, an ethylsulfonyl group, a normal propylsulfonyl group, an isopropylsulfonyl group, a normal butylsulfonyl group, a secondary butylsulfonyl group, a tertiary butylsulfonyl group, a normal pentylsulfonyl group, an isopentylsulfonyl group, a tertiary pentylsulfonyl group, a neopentylsulfonyl group, a 2,3-dimethylpropylsulfonyl group, a 1-ethylpropylsulfonyl group, a 1-methylbutylsulfonyl group, a normal hexylsulfonyl group, an isohexylsulfonyl group, or a 1,1,2-trimethylpropylsulfonyl group.

At substitutable positions of the " (C₁-C₆) alkyl group", " (C₂-C₆)alkenyl group", " (C₂-C₆)alkynyl group", "(C₁-C₆)alkoxy group", "(C₁-C₈)alkylthio group", "(C₁-C₆)alkylsulfinyl group", or "(C₁-C₆)alkylsulfonyl group" described above, one or two or more halogen atoms may be substituted. When two or more halogen atoms are substituted, the halogen atoms are identical or different. The substituted groups are respectively referred to as "halo(C₁-C₆)alkyl group", "halo (C₂-C₆)alkenyl group", "halo(C₂-C₆)alkynyl group", "halo (C₁-C₆)alkoxy group", "halo(C₁-C₆)alkylthio group", "halo(C₁-C₆)alkylsulfinyl group", or "halo (C₁-C₆)alkylsulfonyl group".

Expressions such as "(C₁-C₆)" and "(C₂-C₆)" indicate the range of carbon atoms of various substituents. In addition, the above definitions can be applied to the groups in which the above substituents are linked. For example, the "(C₁-C₆)alkoxy(C₁-C₆)alkyl group" indicates that a linear or branched alkoxy group having 1 to 6 carbon atoms is attached to a linear or branched alkyl group having 1 to 6 carbon atoms.

The " (C₁-C₆) alkoxycarbonyl group" refers to an alkoxycarbonyl group composed of a carbonyl group and a linear or branched alkoxy group having 1 to 6 carbon atoms, such as a methoxycarbonyl group, an ethoxycarbonyl group, a normal propoxycarbonyl group, an isopropoxycarbonyl group, a normal butoxycarbonyl group, a secondary butoxycarbonyl group, a tertiary butoxycarbonyl group, a normal pentyloxycarbonyl group, an isopentyloxycarbonyl group, a tertiary pentyloxycarbonyl group, a neopentyloxycarbonyl group, a 2,3-dimethylpropyloxycarbonyl group, a 1-ethylpropyloxycarbonyl group, a 1-methylbutyloxycarbonyl group, a normal hexyloxycarbonyl group, an isohexyloxycarbonyl group, or a 1,1,2-trimethylpropyloxycarbonyl group.

The "aryl group" refers to an aromatic hydrocarbon group having 6 to 10 carbon atoms, such as a phenyl group, a 1-naphthyl group, or a 2-naphthyl group.

Examples of the salt of the benzimidazole compound represented by the general formula (1) of the present invention include inorganic acid salts such as a hydrochloride, a sulfate, a nitrate, and a phosphate; organic acid salts such as an acetate, a fumarate, a maleate, an oxalate, a methanesulfonate, a benzenesulfonate, a para-tolenesulfonate; and salts with inorganic or organic bases such as a sodium ion, a potassium ion, a calcium ion, and a trimethylammonium.

The benzimidazole compound represented by the general formula (1) or a salt thereof of the present invention may have one asymmetric center in the structural formula, and the present invention encompasses all of the respective optical isomers and mixtures in which they are contained in any proportion. The benzimidazole compound represented by the general formula (1) and a salt thereof of the present invention may also have two geometric isomers derived from a carbon-carbon double bond in the structural formula, and the present invention also encompasses all of the respective geometric isomers and mixtures in which they are contained in any proportion. Furthermore, the benzimidazole compound represented by the general formula (1) or a salt thereof of the present invention may have tautomers in the structural formula, and the present invention also encompasses all of the respective tautomers and mixtures in which they are contained in any proportion.

In the benzimidazole compound represented by the general formula (1) or a salt thereof, which is an active ingredient of the canine filariasis control agent of the present invention,
R is preferably (a1) a hydrogen atom; (a2) a (C₁-C₆)alkyl group; (a3) a halo(C₁-C₆)alkyl group; (a4) a (C₂-C₆)alkenyl group; (a5) a (C₂-C₆)alkynyl group; (a6) a (C₁-C₆)alkoxy(C₁-C₆)alkyl group; (a7) a (C₁-C₆)alkylthio(C₁-C₆)alkyl group; (a8) a (C₁-C₆)alkoxycarbonyl(C₁-C₆)alkyl group; (a9) a (C₁-C₆)alkoxycarbonyl group; (a10) an aryl (C₁-C₆)alkyl group; (a12) an aryl (C₁-C₆) alkoxy(C₁-C₆)alkyl group; (a15) an R³R⁴N carbonyl group, wherein R³ and R⁴ are the same as above; (a16) an R³R⁴N thiocarbonyl group, wherein R³ and R⁴ are the same as above; or (a17) an R³R⁴N sulfonyl group, wherein R³ and R⁴ are the same as above;
X¹ and X³ are preferably both (b1) a hydrogen atom;
X² is preferably (c1) a (C₁-C₈)alkyl group; (c3) a (C₂-C₈)alkynyl group; (c5) an aryl group having 1 to 5 identical or different substituents selected from (a) a halogen atom, (b) a (C₁-C₆)alkyl group, (c) a halo(C₁-C₆)alkyl group, (d) a (C₁-C₆)alkoxy group, (e) a halo(C₁-C₆)alkoxy group, (f) a (C₁-C₆)alkylthio group, (g) a halo(C₁-C₆)alkylthio group, (h) a (C₁-C₆)alkylsulfinyl group, (i) a halo(C₁-C₆)alkylsulfinyl group, (1) a nitro group, and (m) a trimethylsilyl group; or (c7) an aryl(C₁-C₆)alkyl group having 1 to 5 identical or different substituents selected from (a) a halogen atom, (b) a (C₁-C₆)alkyl group, (c) a halo(C₁-C₆)alkyl group, (d) a (C₁-C₆)alkoxy group, (e) a halo(C₁-C₆)alkoxy group, (f) a (C₁-C₆)alkylthio group, (g) a halo(C₁-C₆)alkylthio group, (h) a (C₁-C₆)alkylsulfinyl group, (i) a halo(C₁-C₆)alkylsulfinyl group, (j) a (C₁-C₆)alkylsulfonyl group, (k) a halo (C₁-C₆) alkylsulfonyl group, (l) a nitro group, and (m) a trimethylsilyl group;
Y¹ and Y⁴ are identical or different, and are preferably (d1) a hydrogen atom, (d2) a halogen atom, or (d3) a (C₁-C₆)alkyl group;
Y² and Y³ are identical or different, and are preferably (e1) a hydrogen atom; (e2) a halogen atom; (e3) a (C₁-C₆)alkyl group; (e5) a (C₁-C₆) alkylthio group; (e6) a halo (C₁-C₆) alkyl group; (e7) a halo (C₁-C₆) alkoxy group; (e8) an aryloxy group; (e10) an arylthio group; or (e12) an arylcarbonyl group.

The benzimidazole compounds represented by general formulae (1) or salts thereof, which are the active ingredients of the canine filariasis control agent of the present invention can be produced, for example, by the following production methods. However, the present invention is not limited thereto.

### Production method 1

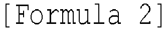

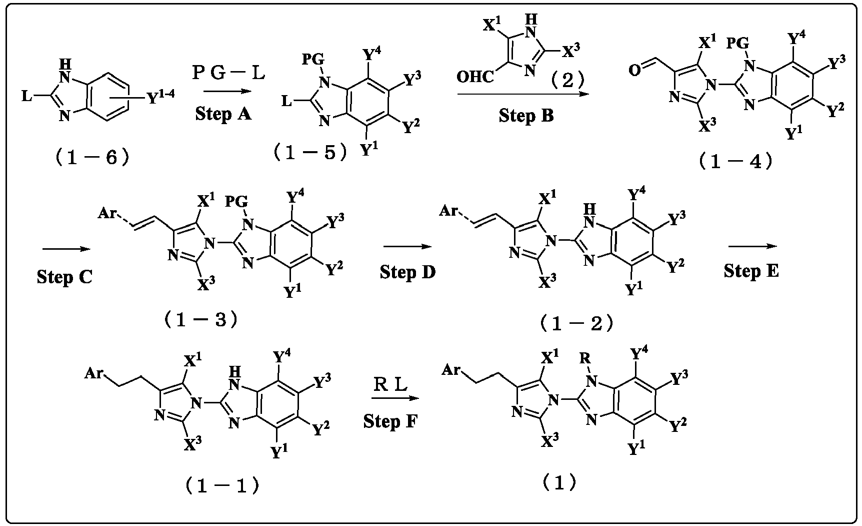

wherein R, X¹, X³, Y¹, Y², Y³ and Y⁴ are the same as above, Ar represents an aryl group, L represents a leaving group, and PG represents a protecting group.

### Production method of step [A]

The benzimidazole compound represented by general formula (1-5) can be produced by reacting the benzimidazole compound represented by general formula (1-6), which is easily available, with a compound represented by PG-L, for example, a protective agent such as methoxymethyl chloride (MOMCl), methoxyethoxymethyl chloride (MEMCl), or 2-(trimethylsilyl)ethoxymethyl chloride (SEMCl), in the presence of an inert solvent and a base.

Examples of the base used in this reaction include inorganic bases such as sodium hydroxide, potassium hydroxide, sodium carbonate, potassium carbonate, sodium bicarbonate, potassium bicarbonate, sodium hydride, and potassium hydride; acetates such as sodium acetate and potassium acetate; alkali metal alkoxides such as potassium t-butoxide, sodium methoxide, and sodium ethoxide; tertiary amines such as triethylamine, diisopropylethylamine, and 1,8-diazabicyclo[5.4.0]undec-7-ene (DBU), and nitrogen-containing aromatic compounds such as pyridine and dimethylaminopyridine (DMAP). The amount of the base used is typically in the range of 1-fold mole to 10-fold moles of the compound represented by the general formula (1-6).

The inert solvent used in this reaction may be any inert solvent not significantly inhibit the progress of this reaction, and examples thereof include aromatic hydrocarbons such as benzene, toluene, and xylene; halogenated hydrocarbons such as methylene chloride, chloroform, and carbon tetrachloride; halogenated aromatic hydrocarbons such as chlorobenzene and dichlorobenzene; linear or cyclic ethers such as diethyl ether, methyl tertiary butyl ether, dioxane, and tetrahydrofuran (THF); esters such as ethyl acetate; amides such as dimethylformamide (DMF), dimethylacetamide (DMA), and N-methylpyrrolidone (NMP); ketones such as acetone and methyl ethyl ketone; polar solvents such as dimethyl sulfoxide (DMSO) and 1,3-dimethyl-2-imidazolidinone (DMI); and nitrogen-containing aromatic compounds such as pyridine. These inert solvents can be used singly or in combination of two or more.

Since this reaction is an equimolar reaction, each reactant may be used in equimolar, but either reactant may also be used in excess. The reaction temperature can be in the range of room temperature to the boiling point of the inert solvent used. The reaction time may vary depending on the reaction scale and reaction temperature but may be in the range of several minutes to 48 hours.

After the reaction is completed, the target compound can be isolated from the reaction system containing the target compound by conventional methods, and the target compound can be prepared by purifying as needed by recrystallization, column chromatography, or the like. The next step may be performed without isolating the intermediate from the reaction system.

### Production method of step [B]

The benzimidazole compound represented by general formula (1-4) can be produced by reacting the benzimidazole compound represented by the general formula (1-5) with the substituted imidazole compound represented by general formula (2) in the presence of an inert solvent and a base.

Examples of the base used in this reaction include inorganic bases such as sodium hydroxide, potassium hydroxide, sodium carbonate, potassium carbonate, sodium bicarbonate, potassium bicarbonate, and sodium hydride; acetates such as sodium acetate and potassium acetate; alkali metal alkoxides such as potassium t-butoxide, sodium methoxide, and sodium ethoxide; tertiary amines such as triethylamine, diisopropylethylamine, and 1,8-diazabicyclo[5.4.0]undec-7-ene (DBU); and nitrogen-containing aromatic compounds such as pyridine and dimethylaminopyridine (DMAP). The amount of the base used is typically in the range of 1-fold mole to 10-fold moles of the compound represented by the general formula (2) .

The inert solvent used in this reaction may be any inert solvent not significantly inhibit this reaction, and examples thereof include aromatic hydrocarbons such as benzene, toluene, and xylene; halogenated hydrocarbons such as methylene chloride, chloroform, and carbon tetrachloride; halogenated aromatic hydrocarbons such as chlorobenzene and dichlorobenzene; linear or cyclic ethers such as diethyl ether, tetrahydrofuran (THF), and dioxane; and amides such as dimethylformamide (DMF), dimethylacetamide (DMA), and N-methylpyrrolidone (NMP). These inert solvents can also be used singly or in combination of two or more.

Since this reaction is an equimolar reaction, each reactant may be used in equimolar, but either reactant may also be used in excess. The reaction temperature can be in the range of room temperature to the boiling point of the inert solvent used. The reaction time may vary depending on the reaction scale and reaction temperature but may be in the range of several minutes to 48 hours.

After the reaction is completed, the target compound can be isolated from the reaction system containing the target compound by conventional methods, and the target compound can be prepared by purifying as needed by recrystallization, column chromatography, or the like. The next step may be performed without isolating the intermediate from the reaction system.

### Production method of step [C]

The benzimidazole compound represented by general formula (1-3) can be produced by reacting the benzimidazole compound represented by the general formula (1-4) with a phosphonium salt produced by the reaction of triphenylphosphine and arylalkyl halide, the so-called Wittig reagent, in the presence of an inert solvent.

Examples of the base used in this reaction include inorganic bases such as sodium hydride, potassium hydroxide, sodium hydroxide, potassium hydroxide, sodium carbonate, potassium carbonate, sodium bicarbonate, and potassium bicarbonate. The amount of the base used is typically in the range of 1-fold mole to 10-fold moles of the compound represented by the general formula (1-4).

The inert solvent used in this reaction may be any inert solvent not significantly inhibit this reaction, and examples thereof include aromatic hydrocarbons such as benzene, toluene, and xylene; halogenated aromatic hydrocarbons such as chlorobenzene and dichlorobenzene; and linear or cyclic ethers such as diethyl ether, tetrahydrofuran (THF), and dioxane. These inert solvents can also be used singly or in combination of two or more.

Since this reaction is an equimolar reaction, each reactant may be used in equimolar, but either reactant may also be used in excess. The reaction temperature in this reaction may be typically in the range of about 0°C to the boiling point of the solvent used. The reaction time may vary depending on the reaction scale, reaction temperature, or the like, and may not be constant, but may be appropriately selected from the range of several minutes to 48 hours.

After the reaction is completed, the target can be isolated by conventional methods, and the target can be prepared by purifying as needed by recrystallization, distillation, or the like. The next step may be performed without isolating the intermediate from the reaction system.

### Production method of step [D]

The benzimidazole compound represented by general formula (1-2) can be produced by removing the protective group of the benzimidazole compound represented by the general formula (1-3) according to the method described in Greene's PROTECTIVE GROUPS in ORGANIC SYNTHESIS(4th Edition)561-565, or the like.

### Production method of step [E]

The benzimidazole compound represented by general formula (1-1) can be produced from the benzimidazole compound represented by the general formula (1-2) in the presence of a reducing catalyst, under a hydrogen atmosphere, under atmospheric pressure or under pressure, in the presence or absence of an inert solvent.

Examples of the reducing catalyst include palladium catalysts such as palladium(0)/carbon and palladium hydroxide/carbon, and nickel catalysts such as Raney nickel. The amount of the catalyst used may be appropriately selected from the range of 0.001 molar equivalent to 0.5 molar equivalent of the benzimidazole compound represented by the general formula (1-2). The reaction temperature may be appropriately selected from the range of -20°C to the reflux temperature of the solvent used. The reaction time may vary depending on the reaction scale, but may be appropriately selected from the range of several minutes to 100 hours.

The reaction pressure may be appropriately selected from the range of 0.1 Mpa to 5 Mpa. The inert solvent may be any inert solvent not significantly inhibit the progress of this reaction, and examples thereof include aliphatic hydrocarbons such as hexane, cyclohexane, and methylcyclohexane; aromatic hydrocarbons such as toluene, benzene, and chlorobenzene; amides such as dimethylformamide and dimethylacetamide; esters such as ethyl acetate and butyl acetate; alcohols such as methanol, ethanol, n-propanol, and i-propanol; organic acids such as 1,3-dimethyl-2-imidazolidinone, acetic acid, and propionic acid; and water. These inert solvents can also be used singly or in combination of two or more.

After the reaction is completed, the target can be isolated from the reaction system containing the target by conventional methods, and the target can be prepared by purifying as needed by recrystallization, column chromatography, distillation, or the like.

### Production method of step [F]

The benzimidazole compound represented by the general formula (1) can be produced by reacting the benzimidazole compound represented by the general formula (1-1) with the compound represented by RL, wherein L represents a leaving group such as a halogen atom, in the presence of an inert solvent and a base.

Examples of the base used in this reaction include inorganic bases such as sodium hydride, sodium hydroxide, potassium hydroxide, sodium carbonate, potassium carbonate, sodium bicarbonate, and potassium bicarbonate; and acetates such as sodium acetate and potassium acetate. The amount of the base used is typically in the range of 1-fold mole to 10-fold moles of the compound represented by the general formula (1-1).

The amount of the compound represented by RL used is typically in the range of 1-fold mole to 10-fold moles of the compound represented by the general formula (1-1).

The inert solvent used in this reaction may be any inert solvent not significantly inhibit this reaction, and examples thereof include aromatic hydrocarbons such as benzene, toluene, and xylene; halogenated hydrocarbons such as methylene chloride, chloroform, and carbon tetrachloride; halogenated aromatic hydrocarbons such as chlorobenzene and dichlorobenzene; and linear or cyclic ethers such as diethyl ether, tetrahydrofuran (THF), and dioxane. These inert solvents can also be used singly or in combination of two or more.

The reaction temperature in this reaction may typically be in the range of about 0°C to the boiling point of the solvent used. The reaction time may vary depending on the reaction scale, reaction temperature, or the like, and may not be constant, but may be appropriately selected from the range of several minutes to 48 hours.

After the reaction is completed, the target compound can be isolated by conventional methods, and the target compound can be prepared by purifying as needed by recrystallization, distillation, or the like.

Next, specific examples of the compounds of the present invention and their intermediates are shown below. In the table below, Me represents a methyl group, Et represents an ethyl group, n-Pro represents a normal propyl group, Ph represents a phenyl group, and Bn represents a benzyl group. Physical properties indicate melting point (°C) or ¹H-NMR. ¹H-NMR data are shown in Tables 4.

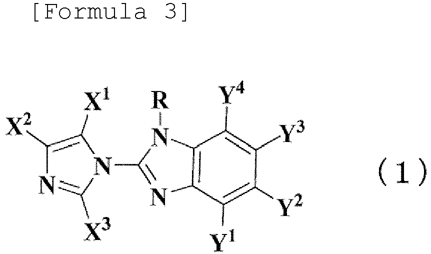

### [Table 1]

**Table 1**

| Compound No. | X¹ | X² | X³ | Y¹ | Y² | Y³ | Y⁴ | R | Physical properties |
|---|---|---|---|---|---|---|---|---|---|
| 1-1 | H | 2,4-Cl₂PhCH₂CH₂ | H | H | H | H | H | H | NMR |
| 1-2 | H | 2,4-Br₂PhCH₂CH₂ | H | H | Br | Br | H | Me | NMR |

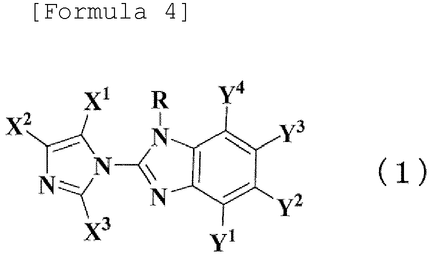

### [Table 2]

**Table 2**

| Compound No. | X² | Y¹ | Y² | Y³ | Y⁴ | R | Physical properties |
|---|---|---|---|---|---|---|---|
| 2-1 | 2,4-Cl₂PhCH₂CH₂ | H | H | COPh | H | H | 210-212 |
| 2-2 | 2,4-Cl₂PhCH₂CH₂ | H | H | SPh | H | H | 82-83 |
| 2-3 | 2,4-Cl₂PhCH₂CH₂ | H | H | S-n-Pro | H | H | 129-130 |
| 2-4 | 2,4-Cl₂PhCH₂CH₂ | H | Cl | Cl | H | H | 217-218 |
| 2-5 | 2,4-Cl₂PhCH₂CH₂ | H | H | I | H | H | 118-119 |
| 2-6 | 2,4-Cl₂PhCH₂CH₂ | H | H | Br | H | H | 184-185 |
| 2-7 | 2,4-Cl₂PhCH₂CH₂ | H | Br | H | Br | H | 131-132 |
| 2-8 | 2,4-Cl₂PhCH₂CH₂ | H | OCF₃ | H | Br | H | 125-126 |
| 2-9 | 2,4-Cl₂PhCH₂CH₂ | H | H | CF₃ | H | H | 107-108 |
| 2-10 | 2,4-Cl₂PhCH₂CH₂ | H | H | OCF₃ | H | H | 97-98 |
| 2-11 | 2,4-Cl₂PhCH₂CH₂ | H | F | F | F | H | 205-206 |
| 2-12 | 2,4-Cl₂PhCH₂CH₂ | H | Br | H | Me | H | 119-120 |
| 2-13 | 2,4-Cl₂PhCH₂CH₂ | H | H | OPh | Cl | H | 204-205 |
| 2-14 | 2,4-Cl₂PhCH₂CH₂ | H | H | Me | Me | H | 95-96 |
| 2-15 | 2-ClPhCH₂CH₂ | H | Br | Br | H | H | 236-237 |
| 2-16 | 2,4-Me₂PhCH₂CH₂ | H | Br | Br | H | H | 110-111 |
| 2-17 | 2-Cl-4-CF₃PhCH₂CH₂ | H | Br | Br | H | H | 130-131 |
| 2-18 | 2,4-Cl₂Ph | H | Br | Br | H | H | 278-280 |
| 2-19 | 3-ClPhCH₂CH₂ | H | Br | Br | H | H | 191-192 |
| 2-20 | 4-ClPhCH₂CH₂ | H | Br | Br | H | H | 114-115 |

| | | | | | | | |
|---|---|---|---|---|---|---|---|
| X¹ and X³ represent a hydrogen atom. | | | | | | | |

### [Table 3]

**Table 3**

| Compound No. | X² | Y² | Y³ | Y⁴ | R | Physical properties |
|---|---|---|---|---|---|---|
| 2-21 | 2,3-Cl₂PhCH₂CH₂ | Br | Br | H | H | 247-248 |
| 2-22 | 2,5-Cl₂PhCH₂CH₂ | Br | Br | H | H | 129-130 |
| 2-23 | 2,6-Cl₂PhCH₂CH₂ | Br | Br | H | H | 289-290 |
| 2-24 | 3,4-Cl₂PhCH₂CH₂ | Br | Br | H | H | 124-125 |
| 2-25 | 3,5-Cl₂PhCH₂CH₂ | Br | Br | H | H | 140-141 |
| 2-26 | 2,4-Br₂PhCH₂CH₂ | Br | Br | H | H | 131-132 |
| 2-27 | n-Bu-≡ | H | H | H | H | |
| 2-28 | n-Hex | H | H | H | H | |
| 2-29 | n-Hex | H | H | H | Me | |
| 2-30 | n-Hex | H | H | H | allyl | |
| 2-31 | n-Hex | H | H | H | propargyl | |
| 2-32 | n-Hex | H | H | H | Bn | |
| 2-33 | n-Hex | H | H | H | CH₂OCH₃ | |
| 2-34 | n-Hex | H | H | H | CH₂OBn | |
| 2-35 | n-Hex | H | H | H | CH₂SCH₃ | |
| 2-36 | n-Hex | H | H | H | CH₂CF₃ | |
| 2-37 | n-Hex | H | H | H | CH₂CO₂CH₃ | |
| 2-38 | n-Hex | H | H | H | CO₂Et | |
| 2-39 | n-Hex | H | H | H | CONMe₂ | |
| 2-40 | n-Hex | H | H | H | CSNMe₂ | |
| 2-41 | n-Hex | H | H | H | SO₂NMe₂ | |

| | | | | | | |
|---|---|---|---|---|---|---|
| X¹, X³, and Y¹ represent a hydrogen atom. | | | | | | |

### [Table 4]

**Table 4**

| Compound No. | ¹H-NMR data(CDCl₃) |
|---|---|
| 1-1 | 11.60(bs, 1H), 7.78-7.34(m, 3H), 7.34-7.24(m, 3H), 7.14-6.97(m, 3H), 3.11-2.99(m, 2H), 2.97-2.86(m, 2H) |
| 1-2 | 8.02 (s, 1H), 7.97 (d, 1H), 7.70 (d, 1H), 7.69 (s, 1H), 7.33 (dd, 1H), 7.06 (d, 2H), 3.73 (s, 3H), 3.12 (t, 2H), 2.96 (t, 2H) |

The canine filariasis control agent according to the present invention contains a benzimidazole compound represented by General Formula (1) or a salt of the compound as an active ingredient.

Non-limiting examples of the animal to which the canine filariasis control agent according to the present invention is applied or administered include domestic animals such as cattle, pigs, rabbits, and birds; and pets such as dogs, rabbits, and cats (hereinafter these are generically referred to as a "subject animal", where the subject animal excludes humans). The subject animal is preferably selected from dogs. However, the subject animal is not limited to the above ones. As used herein, the term "control" refers to and includes prophylaxis and treatment.

Canine filariasis is known to be caused by filariae of Spirurida. Non-limiting detailed examples of the filariae include (a) filariae of Onchocercidae such as Brugia spp. including *Brugia malayi, Brugia pahangi,* and *Brugia patei*; Dipetalonema spp. including *Dipetalonema reconditum*; Dirofilaria spp. including *Dirofilaria immitis*; Filaria spp. including *Filaria oculi;* Onchocerca spp. including *Onchocerca cervicalis, Onchocerca gibsoni,* and *Onchocerca gutturosa*; (b) filariae of Setariidae such as Setaria spp. including *Setaria digitata, Setaria equina, Setaria labiatopapillosa,* and *Setaria marshalli*; Wuchereria spp. including *Wuchereria bancrofti*; (c) filariae of Filariidae such as Parafilaria spp. including *Parafilaria multipapillosa*; Stephanofilaria spp. including *Stephanofilaria assamensis, Stephanofilaria dedoesi, Stephanofilaria kaeli, Stephanofilaria okinawaensis,* and *Stephanofilaria stilesi.*

Upon use of the canine filariasis control agent according to the present invention, the benzimidazole compound may be used as intact without addition of any other components. In general, however, the benzimidazole compound or a salt of the compound is preferably combined with an excipient such as a solid carrier or liquid carrier, and formulated into any of preparations such as tablets, powders, granules, capsules, water-soluble powders, liquids, wettable powders (water-dispersible powders), and suspensions before use, in accordance with a common technique (such as the technique described in "Textbook of Pharmaceutics", edited by OTSUKA, Akinobu et al., 1995, Nankodo Co., Ltd.). Non-limiting examples of the excipients as solid carriers include lactose, sucrose, glucose, corn starch, gelatin, casein, starch, gum arabic, cellulose derivatives, and alginic acid. Non-limiting examples of the excipients as liquid carriers include water, glycerol, vegetable oils, fatty acids, fatty acid esters, and sorbitol.

The canine filariasis control agent according to the present invention may further contain any of other components exemplified by, but not limited to, organic minerals such as peptide-zinc and peptide-iron; inorganic minerals such as zinc carbonate, manganese carbonate, iron sulfate, and magnesium carbonate; vitamins such as vitamin A, vitamin B, vitamin C, vitamin D, vitamin E, vitamin K, folic acid, pantothenic acid, and nicotinic acid; alfalfa meal; and flakes of corn. For higher palatability, a flavor, for example, may be fed simultaneously. Where necessary, the canine filariasis control agent may further contain any of common additives such as antibacterial agents, antifungal agents, anthelmintics, antioxidants, coloring agents, flavoring agents, gustatory substances, and enzymes. The canine filariasis control agent is preferably used after being formulated into any of preparations such as powders, granules, liquids, and tablets, by a common technique. These preparations may each contain, as an active ingredient, the benzimidazole compound or a salt of the compound typically in an amount from about 0.01 to about 95 weight percent (weight ratio).

The canine filariasis control agent according to the present invention, which is formulated in the above manner, can be used as intact or after dilution typically with water. In addition or alternatively, any of other materials may be used in combination as mixing with the canine filariasis control agent, or used simultaneously with, or non-simultaneously with, the canine filariasis control agent. Non-limiting examples of the other materials include common additives such as antibacterial agents, antifungal agents, anthelmintics, antioxidants, coloring agents, flavoring agents, gustatory substances, and enzymes.

The administration technique of the canine filariasis control agent according to the present invention to the subject animal is not limited, and may be a known technique such as scattering on, or mixing with a feed, as described later. The dose is an effective amount for canine filariasis control efficacy on the subject animal, namely, such an amount that the administration of the canine filariasis control agent according to the present invention increases the canine filariasis control efficacy on the subject animal, as compared with the case where the canine filariasis control agent is not administered, provided that the other conditions are identical.

A non-limiting example of the administration technique is the technique of mixing or gelating an active ingredient of the canine filariasis control agent according to the present invention to be administered to the subject animal, and letting the subject animal freely take the mixed or gelated substance. This technique can be performed in any of a hatchery and a farm. The technique can also be performed during transportation of the subject animal from a hatchery to a farm.

Another non-limiting example of the administration technique performable herein is the technique of adding a predetermined amount of a water-soluble polysaccharide powder to the active ingredient of the canine filariasis control agent according to the present invention to give a geletable preparation, diluting the preparation with water to give a gelatinous solid upon use typically in a hatchery or a farm, and administering the gelatinous solid to the subject animal (namely, free feeding or direct administration into the crop).

The formulated canine filariasis control agent is generally used alone, but can be used as a diluted preparation after being diluted with water (namely, administration as a dilution in drinking water). The diluted preparation contains the active ingredient(s) in a concentration of generally preferably from about 10 to 10000 ppm, and more preferably from about 35 to 5000 ppm. The diluted preparation may be administered typically by a technique of dissolving about 0.01 to about 500 g of the canine filariasis control agent in 1 L of water, and dividing the solution to a dose to be administered. Preferably, about 0.035 to about 350 g of the canine filariasis control agent is dissolved in 1 L of water, before administration.

The diluted preparation prepared by the above procedure may be administered to the subject animal typically using a device to add the diluted preparation to drinking water. The administration amount (liquid amount) of the diluted preparation may be determined appropriately according typically to the size, growth situation, and rearing density of the subject animal, and administration technique, but is generally preferably about 300 to about 2000 liters per 10000 subjects.

In the case of egg-laying birds and meat animals, the canine filariasis control agent according to the present invention may be administered continuously over an entire period of rearing of the subject animal, and is preferably administered in a chick stage (e.g., a chicken of 0 to 5 weeks old after hatching) or an animal of 0 to 5 weeks old after birth.

The dose of the canine filariasis control agent may be determined appropriately according typically to the type and size of the subject animal, but is preferably from 0.005 to 2 g, and more preferably from 0.005 to 1 g, in terms of total dose.

The canine filariasis control agent composition according to the present invention is prepared by adding the canine filariasis control agent typically to a feed, or drinking water, or a physiological electrolyte solution for animals. The amount of the canine filariasis control agent to be added is preferably from about 0.005 to about 10.0 weight percent of the totality of the canine filariasis control agent composition.

The feed or drinking water or physiological electrolyte solution for the subject animal, for use in the canine filariasis control agent composition according to the present invention, can be any one that is commonly used and is not limited. Non-limiting examples thereof include feeds prepared by appropriately mixing components. Non-limiting examples of the components include maize (corn), rice, wheat or barley, milo, soybean cake, wheat bran, defatted rice bran, fish meal, skimmed milk powder, dried whey, fats and oils, alfalfa meal, white fish meal, soybean oil, powdered purified beef tallow, wheat flour, rapeseed oil, meat-and-bone meal (feather meal), animal fats and oils, calcium phosphate, corn gluten meal, molasses (syrup), corn germ meal, calcium carbonate, tricalcium phosphate, sodium chloride, choline chloride, vitamins (such as vitamin A, vitamin B1, vitamin B2, vitamin B6, vitamin B12, vitamin D, vitamin E, calcium pantothenate, nicotinamide, and folic acid), amino acids (such as lysine and methionine), trace inorganic salts (such as magnesium sulfate, iron sulfate, copper sulfate, zinc sulfate, potassium iodide, and cobalt sulfate), and attenuated vaccines.

The canine filariasis control agent composition according to the present invention may further contain any of other components. Non-limiting examples of such other components include organic minerals such as peptide-zinc and peptide-iron; inorganic minerals such as zinc carbonate, manganese carbonate, iron sulfate, and magnesium carbonate; vitamins such as vitamin A, vitamin B, vitamin C, vitamin D, vitamin E, vitamin K, folic acid, pantothenic acid, and nicotinic acid; alfalfa meal; and flakes of corn. For higher palatability, a flavor, for example, may be fed simultaneously.

The administration technique of the canine filariasis control agent composition according to the present invention to the subject animal is not limited, and may be an appropriate administration or feeding technique using a process such as scattering on, or mixing with a feed, as described later. The dose of the canine filariasis control agent composition is, in short, an effective amount for canine filariasis control efficacy on the subject animal, namely, such an amount that the administration of the canine filariasis control agent composition according to the present invention increases the canine filariasis control efficacy on the subject animal, as compared with the case where the canine filariasis control agent composition is not administered, provided that the other conditions are identical.

In the case of egg-laying birds and meat animals, the canine filariasis control agent composition according to the present invention may be administered continuously over an entire period of rearing of the subject animal, and is preferably administered in a chick stage (e.g., a chicken of 0 to 5 weeks old after hatching) or an animal of 0 to 5 weeks old after birth, and is more preferably administered continuously from 0 to 21 days after hatching or from 0 to 21 days after birth.

When incorporated into a subject animal feed, the canine filariasis control agent composition according to the present invention may be used in such an amount that the benzimidazole compound or a salt of the compound is present in a proportion from about 0.0005 to about 5 weight percent, and preferably from about 0.05 to about 2 weight percent. When added to drinking water or a physiological electrolyte solution before use, the canine filariasis control agent composition may be used in such an amount that the benzimidazole compound or a salt of the compound is present in a proportion from about 0.035 to about 3.5 weight percent, and preferably from about 0.035 to about 1.4 weight percent.

A control method according to an embodiment of the present invention includes the step of administering, to a subject animal, an effective amount of the canine filariasis control agent according to the present invention or the canine filariasis control agent composition according to the present invention. In the method, the canine filariasis control agent composition can be administered or fed to the animal by a common technique. The effective amount varies depending on conditions such as the type of each preparation, the subject animal, and the intake time period, can be selected appropriately regardless of the ranges, and can be higher than, or lower than, the above-mentioned ranges.

Specifically, for example, the canine filariasis control agent according to the present invention is diluted with water to a concentration suitable for the administration to the subject animal, and the diluted agent is administered to the subject animal. The dilution degree can be determined in accordance with conventional drinking water dilution administration techniques, and is preferably from about 5 to about 10 times to give a diluted agent. Alternatively, a gelatinous solid may be administered to the subject animal. The gelatinous solid is prepared in the following manner. The canine filariasis control agent according to the present invention is diluted with water to a predetermined concentration, this is combined with a water-soluble polysaccharide with stirring to give a homogeneous solution, and the solution stands at room temperature or is stored in a cold place (such as a refrigerator), to give the gelatinous solid. Alternatively, the gelatinous solid may be prepared using a gelling agent that melts at a high temperature and solidifies at a low temperature (such as agar or gelatin). In this case, the gelling agent is previously added to a medium for preparing the canine filariasis control agent according to the present invention. The medium is cooled after high-pressure steam sterilization and stands at room temperature or is stored in a cold place (such as a refrigerator), to give the gelatinous solid. In the gelation, the gel strength is appropriately from about 200 to about 2000 g/cm². Assume that an agar is used as the gelling agent. In this case, the gel strength in the range corresponds to an agar concentration of about 0.5% to about 3.0%, although the agar concentration may vary depending on the type of agar to be used.

Non-limiting examples of the polysaccharide used for the gelation of the canine filariasis control agent according to the present invention in the aqueous medium include agar, carrageenan, carboxymethylcellulose, starch, mannan, gelatin, sodium alginate, gum arabic, locust bean gum, xanthan gum, chitosan, guar gum, pectin, propyl alginate glycol ester, arabinogalactan, ghatti gum, tamarind seed gum, pullulan, morpholine fatty acid salts, curdlan, and gum traganth (gum tragacanth). Among these polysaccharides, agar, starch, mannan, or gelatin is particularly preferably employed, because of non-expensive, easy availability.

For example, when the gelatinous solid is administered to a poultry, the gelatinous solid may be administered to an about 0 to 7 day-old poultry, which takes in small amounts of drinking water and feed. This technique enables feeding of a necessary amount of the canine filariasis control agent according to the present invention to the poultry for a short time in a laborsaving manner, because the poultry has a genetic program (habit) of pecking a solid on the floor to take the solid in. According to this technique, a material that is hardly administrable to such a young poultry as above can also be efficiently administered to the poultry together with the canine filariasis control agent according to the present invention. This can be done by mixing the material with the canine filariasis control agent according to the present invention and gelating the mixture with a water-soluble polysaccharide. Non-limiting examples of the material include attenuated vaccines, vaccines, drugs, and nutrients. During the stage of chick (young chicken), feeding of water and nutrients much significantly affects the productivity thereafter. When the nutrients are administered simultaneously with the canine filariasis control agent, non-limiting examples of the nutrients include carbohydrates exemplified by saccharides such as glucose, mannose, fructose, and other monosaccharides and oligomers thereof, and sucrose and other disaccharides; proteins such as skimmed milk; and lipids; as well as vitamins, and minerals.

The canine filariasis control agent according to the present invention may be used in combination with any of existing canine filariasis control agents, to strengthen or supplement its effects or efficacies. In such a combination use, two or more active ingredients may be mixed and formulated into a single preparation before administration, or two or more different preparations may be prepared and administered separately.

### Examples

The present invention will be illustrated in further detail with reference to production examples, formulation example, and a test example below. It should be noted, however, the examples are by no means intended to limit the scope of the present invention.

### Reference Example 1. Method for producing 1-methoxymethyl-2-(4-formyl-1H-imidazol-1-yl)benzimidazole

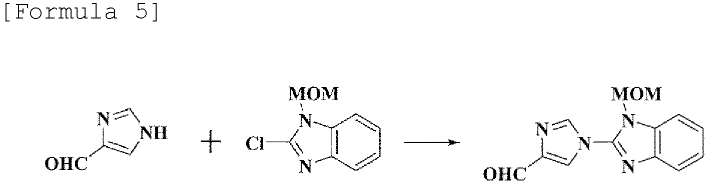

1-Methoxymethyl-2-chlorobenzimidazole (10 g, 50 mmol), 4-formyl-1H-imidazole (5.5 g, 1.1 eq.), and potassium carbonate (21 g, 3 eq.) were added to NMP (50 mL), and the mixture was reacted at 120°C for 3 hours. After the resultant was cooled to room temperature, water was added, then the mixture was extracted with ethyl acetate. The organic layer was washed with saturated saline and dried over sodium sulfate. After the solvent was distilled off, the residue was purified by column chromatography to obtain the target (6.0 g, 47% yield).

### Reference Example 2. Method for producing 1-methoxymethyl-2-(4-(2-(2,4-dichlorophenyl)vinyl)-1H-imidazol-1-yl)benzimidazole

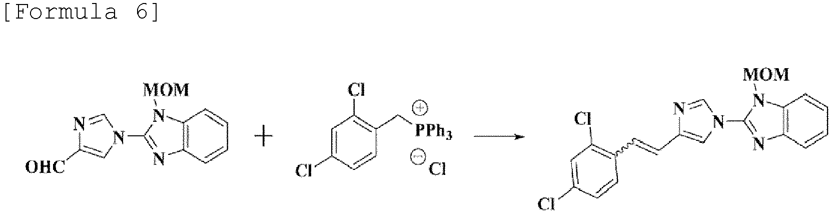

1-Methoxymethyl-2-(4-formyl-1H-imidazol-1-yl)benzimidazole (1.0 g, 4.0 mmol) was added to THF (20 mL), and 2,4-dichlorobenzyltriphenylphosphonium chloride (2.2 g, 1.2 eq.) and 60% sodium hydride (0.24 g, 1.5 eq.) were added. Then, the mixture was warmed to 50°C and reacted for 2 hours. After the resultant was cooled to room temperature, water was added to stop the reaction, and the mixture was extracted with ethyl acetate. The organic layer was washed with a saturated saline solvent and dried over sodium sulfate. The solvent was distilled off to obtain the target (1.3 g, 81% yield).

### Reference Example 3. Method for producing 2-(4-(2-(2,4-dichlorophenyl)vinyl)-1H-imidazol-1-yl)benzimidazole

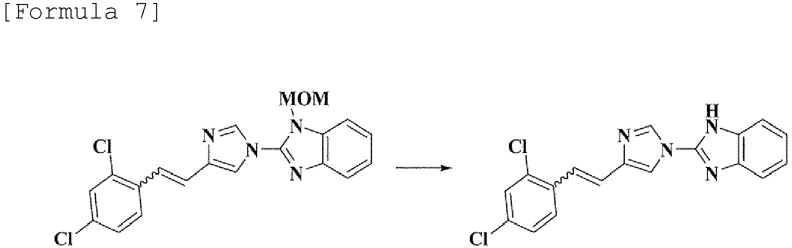

1-Methoxymethyl-2-(4-(2-(2,4-dichlorophenyl)vinyl)-1H-imidazol-1-yl)benzimidazole (1.3 g, 3.2 mmol) was added to THF (20 mL), and concentrated hydrochloric acid (10 mL) was added. The mixture was then reacted under reflux for 2 hours. The resultant was cooled to room temperature, then neutralized with an aqueous potassium hydroxide solution and the mixture was extracted with ethyl acetate. The organic layer was washed with a saturated saline solvent and dried over sodium sulfate. The solvent was distilled off to obtain the target.

### Example 1. Method for producing 2-(4-(2-(2,4-dichlorophenyl)ethyl)-1H-imidazol-1-yl)benzimidazole (Compound No. 1-1)

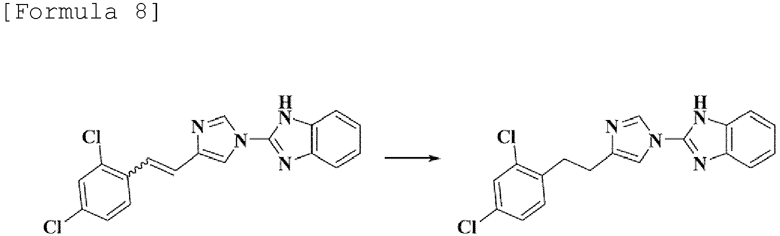

2-(4-(2-(2,4-dichlorophenyl)vinyl)-1H-imidazol-1-yl)benzimidazole obtained in the previous step was added to ethanol (20 mL), then 5% Pd/C (0.34 g) was added. After hydrogen replacement in the reactor, the mixture was reacted at room temperature under a hydrogen atmosphere of 0.4 MPa for 3 hours. The reaction solution was filtered through celite to remove Pd/C, then the filtrate was concentrated to obtain the target (0.35 g, yield 31% (from the previous step)).

### Intermediate production example 1.

### Method for producing 2-chloro-1-methoxymethylbenzimidazole

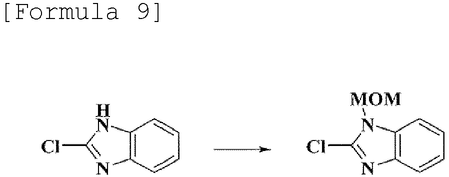

2-Chlorobenzimidazole (10.0 g, 74.6 mmol) was dissolved in DMA (60 mL) and sodium hydride (3.58 g, 1.2 eq.) was added in portions at room temperature. The mixture was reacted for 5 minutes, then chloromethyl methyl ether (MOMCl) (7.8 g, 1.3 eq.) was added at room temperature. The mixture was reacted at room temperature for 3 hours, then the reaction was terminated with water. The mixture was extracted with ethyl acetate, then the organic layer was dried over magnesium sulfate, and the solvent was distilled off. The resulting crude product was purified by column chromatography to obtain the target (13.6 g, 93% yield).

### Formulation Example 1: Powder

In a mortar, 25 parts of a benzimidazole compound and 25 parts of lactose are mixed sufficiently, the mixture is stirred and blended thoroughly, and yields a powder.

### Formulation Example 2: Granules

25 parts of a benzimidazole compound is combined with 25 parts of lactose, followed by stirring and blending thoroughly. Next, the mixture is combined with an appropriate amount of water, further stirred, formed into granules using a granulator, dried by forced air drying, and yields granules.

### Formulation Example 3: Wettable powder

A wettable powder is obtained by uniformly mixing 25 parts of a benzimidazole compound, 65 parts of diatomaceous earth, 5 parts of a higher alcohol sulfuric ester, and 5 parts of an alkylnaphthalenesulfonic acid salt, and pulverizing the mixture finely.

### Test Example 1

### Impact evaluation test on larvae motility of canine filariae (Dirofilaria immitis)

Five hundred L-1 stage larvae of canine filariae diluted in a predetermined prepared solution were inoculated in each well of a 96-well plate, a DMSO dilute solution of the benzimidazole compound represented by the general formula (1) of the present invention or a salt thereof was added to give a final concentration of 50 ppm. Then, the larvae were allowed to stand for 3 days and the mobility ability thereof was investigated. The motility impediment rate of each treatment plot was corrected and calculated based on the impediment efficacy by a DMSO solution alone, and the impact efficacy was evaluated according to the criteria shown below.

### Criteria

A: the corrected motility impediment rate is 100%
B: the corrected motility impediment rate is 99% to 90%
C: the corrected motility impediment rate is 89% to 80%
D: the corrected motility impediment rate is 79% to 50%

### Criteria

As a result, the compound 1-1 of the present invention showed the activity level evaluated as A.

### Test Example 2. Impact evaluation test on growth of canine filariae (Dirofilaria immitis)

To each well of a 24-well plate, a predetermined prepared solution, 8 to 20 L-3 stage larvae of canine filariae, and a DMSO dilute solution of the benzimidazole compound of the present invention were added to give a final concentration of 50 ppm. After seven days, the larvae grown to L-4 stage were counted to calculate the inhibition rate of growth from L3-stage larvae to L4-stage larvae.

As a result, among the compounds represented by the general formula (1) of the present invention, the compounds of Compound Nos. 2-2, 2-9, 2-10, 2-17, 2-18, and 2-26 showed the growth inhibition rate of 50% or more.

### Industrial Applicability

The present invention can provide a canine filariasis control agent for a subject animal, which is administered to the subject animal to exert an excellent effect, and a method of using a canine filariasis control agent for a subject animal using the same.

## Claims

1. A benzimidazole compound represented by general formula (1) 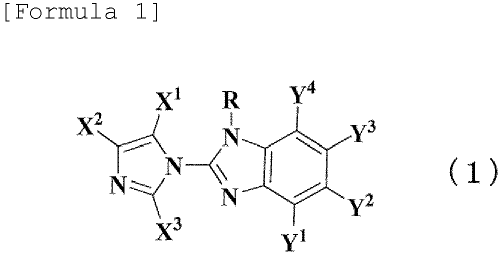 wherein
R represents
(a1) a hydrogen atom;
(a2) a (C₁-C₆) alkyl group;
(a3) a halo (C₁-C₆) alkyl group;
(a4) a (C₂-C₆) alkenyl group;
(a5) a (C₂-C₆)alkynyl group;
(a6) a (C₁-C₆)alkoxy(C₁-C₆)alkyl group;
(a7) a (C₁-C₆) alkylthio (C₁-C₆) alkyl group;
(a8) a (C₁-C₆) alkoxycarbonyl (C₁-C₆) alkyl group;
(a9) a (C₁-C₆)alkoxycarbonyl group;
(a10) an aryl(C₁-C₆)alkyl group;
(a11) an aryl(C₁-C₆)alkyl group having 1 to 5 identical or different substituents selected from (a) a halogen atom, (b) a (C₁-C₆)alkyl group, (c) a halo (C₁-C₆)alkyl group, (d) a (C₁-C₆)alkoxy group, (e) a halo (C₁-C₆)alkoxy group, (f) a (C₁-C₆)alkylthio group, (g) a halo (C₁-C₆)alkylthio group, (h) a (C₁-C₆)alkylsulfinyl group, (i) a halo (C₁-C₆)alkylsulfinyl group, (j) a (C₁-C₆)alkylsulfonyl group, (k) a halo(C₁-C₆)alkylsulfonyl group, (l) a nitro group, and (m) a trimethylsilyl group;
(a12) an aryl (C₁-C₆)alkoxy(C₁-C₆)alkyl group;
(a13) an aryl (C₁-C₆)alkoxy(C₁-C₆)alkyl group having 1 to 5 identical or different substituents selected from (a) a halogen atom, (b) a (C₁-C₆)alkyl group, (c) a halo(C₁-C₆)alkyl group, (d) a (C₁-C₆)alkoxy group, (e) a halo(C₁-C₆)alkoxy group, (f) a (C₁-C₆) alkylthio group, (g) a halo (C₁-C₆)alkylthio group, (h) a (C₁-C₆)alkylsulfinyl group, (i) a halo(C₁-C₆)alkylsulfinyl group, (j) a (C₁-C₆)alkylsulfonyl group, (k) a halo (C₁-C₆)alkylsulfonyl group, (l) a nitro group, and (m) a trimethylsilyl group;
(a14) an R³R⁴N group, wherein R³ and R⁴ are identical or different, and represent (aa) a hydrogen atom, (ab) a (C₁-C₆)alkyl group, (ac) a (C₁-C₆)alkoxy group, (ad) a (C₁-C₆)alkoxycarbonyl group, (ae) a (C₁-C₆)alkylsulfonyl group, (af) a halo (C₁-C₆)alkylsulfonyl group, or (ag) a phenyl group;
(a15) an R³R⁴N carbonyl group, wherein R³ and R⁴ are the same as above;
(a16) an R³R⁴N thiocarbonyl group, wherein R³ and R⁴ are the same as above; or
(a17) an R³R⁴N sulfonyl group, wherein R³ and R⁴ are the same as above;
X¹ and X³ are identical or different, and represent
(b1) a hydrogen atom;
(b2) a halogen atom; or
(b3) a (C₁-C₆)alkyl group;
X² represents
(c1) a (C₁-C₈)alkyl group;
(c2) a (C₂-C₈)alkenyl group;
(c3) a (C₂-C₈)alkynyl group;
(c4) an aryl group;
(c5) an aryl group having 1 to 5 identical or different substituents selected from (a) a halogen atom, (b) a (C₁-C₆)alkyl group, (c) a halo (C₁-C₆) alkyl group, (d) a (C₁-C₆)alkoxy group, (e) a halo (C₁-C₆) alkoxy group, (f) a (C₁-C₆)alkylthio group, (g) a halo (C₁-C₆) alkylthio group, (h) a (C₁-C₆)alkylsulfinyl group, (i) a halo (C₁-C₆)alkylsulfinyl group, (1) a nitro group, and (m) a trimethylsilyl group;
(c6) an aryl (C₁-C₆) alkyl group; or
(c7) an aryl(C₁-C₆)alkyl group having 1 to 5 identical or different substituents selected from (a) a halogen atom, (b) a (C₁-C₆) alkyl group, (c) a halo (C₁-C₆) alkyl group, (d) a (C₁-C₆) alkoxy group, (e) a halo (C₁-C₆) alkoxy group, (f) a (C₁-C₆) alkylthio group, (g) a halo (C₁-C₆) alkylthio group, (h) a (C₁-C₆) alkylsulfinyl group, (i) a halo (C₁-C₆)alkylsulfinyl group, (j) a (C₁-C₆) alkylsulfonyl group, (k) a halo (C₁-C₆) alkylsulfonyl group, (1) a nitro group, and (m) a trimethylsilyl group;
Y¹ and Y⁴ are identical or different, and represent
(d1) a hydrogen atom;
(d2) a halogen atom; or
(d3) a (C₁-C₆)alkyl group; and
Y² and Y³ are identical or different, and represent
(e1) a hydrogen atom;
(e2) a halogen atom;
(e3) a (C₁-C₆)alkyl group;
(e4) a (C₁-C₆)alkoxy group;
(e5) a (C₁-C₆)alkylthio group;
(e6) a halo (C₁-C₆) alkyl group;
(e7) a halo (C₁-C₆) alkoxy group;
(e8) an aryloxy group;
(e9) an aryloxy group having 1 to 5 identical or different substituents selected from (a) a halogen atom, (b) a (C₁-C₆) alkyl group, (c) a halo (C₁-C₆) alkyl group, (d) a (C₁-C₆) alkoxy group, (e) a halo (C₁-C₆) alkoxy group, (f) a (C₁-C₆) alkylthio group, (g) a halo (C₁-C₆) alkylthio group, (h) a (C₁-C₆) alkylsulfinyl group, (i) a halo (C₁-C₆)alkylsulfinyl group, (j) a (C₁-C₆) alkylsulfonyl group, (k) a halo (C₁-C₆) alkylsulfonyl group, (1) a nitro group, and (m) a trimethylsilyl group;
(e10) an arylthio group;
(e11) an arylthio group having 1 to 5 identical or different substituents selected from (a) a halogen atom, (b) a (C₁-C₆) alkyl group, (c) a halo (C₁-C₆) alkyl group, (d) a (C₁-C₆) alkoxy group, (e) a halo (C₁-C₆) alkoxy group, (f) a (C₁-C₆) alkylthio group, (g) a halo (C₁-C₆) alkylthio group, (h) a (C₁-C₆)alkylsulfinyl group, (i) a halo(C₁-C₆)alkylsulfinyl group, (j) a (C₁-C₆) alkylsulfonyl group, (k) a halo (C₁-C₆) alkylsulfonyl group, (1) a nitro group, and (m) a trimethylsilyl group;
(e12) an arylcarbonyl group; or
(e13) an arylcarbonyl group having 1 to 5 identical or different substituents selected from (a) a halogen atom, (b) a (C₁-C₆) alkyl group, (c) a halo (C₁-C₆) alkyl group, (d) a (C₁-C₆) alkoxy group, (e) a halo (C₁-C₆) alkoxy group, (f) a (C₁-C₆) alkylthio group, (g) a halo (C₁-C₆) alkylthio group, (h) a (C₁-C₆) alkylsulfinyl group, (i) a halo (C₁-C₆)alkylsulfinyl group, (j) a (C₁-C₆) alkylsulfonyl group, (k) a halo (C₁-C₆) alkylsulfonyl group, (1) a nitro group, and (m) a trimethylsilyl group,
or a salt thereof.

2. The benzimidazole compound according to claim 1 or a salt thereof, wherein R represents
(a1) a hydrogen atom;
(a2) a (C₁-C₆)alkyl group;
(a3) a halo (C₁-C₆) alkyl group;
(a4) a (C₂-C₆) alkenyl group;
(a5) a (C₂-C₆)alkynyl group;
(a6) a (C₁-C₆)alkoxy(C₁-C₆)alkyl group;
(a7) a (C₁-C₆) alkylthio (C₁-C₆) alkyl group;
(a8) a (C₁-C₆) alkoxycarbonyl (C₁-C₆) alkyl group;
(a9) a (C₁-C₆)alkoxycarbonyl group;
(a10) an aryl (C₁-C₆) alkyl group;
(a12) an aryl (C₁-C₆) alkoxy(C₁-C₆) alkyl group;
(a15) an R³R⁴N carbonyl group, wherein R³ and R⁴ are the same as above;
(a16) an R³R⁴N thiocarbonyl group, wherein R³ and R⁴ are the same as above; or
(al7) an R³R⁴N sulfonyl group, wherein R³ and R⁴ are the same as above;
X¹ and X³ are both represent (b1) a hydrogen atom;
X² represents
(c1) a (C₁-C₈)alkyl group;
(c3) a (C₂-C₈)alkynyl group;
(c5) an aryl group having 1 to 5 identical or different substituents selected from (a) a halogen atom, (b) a (C₁-C₆)alkyl group, (c) a halo (C₁-C₆) alkyl group, (d) a (C₁-C₆)alkoxy group, (e) a halo (C₁-C₆) alkoxy group, (f) a (C₁-C₆)alkylthio group, (g) a halo (C₁-C₆) alkylthio group, (h) a (C₁-C₆)alkylsulfinyl group, (i) a halo (C₁-C₆)alkylsulfinyl group, (1) a nitro group, and (m) a trimethylsilyl group; or
(c7) an aryl(C₁-C₆)alkyl group having 1 to 5 identical or different substituents selected from (a) a halogen atom, (b) a (C₁-C₆) alkyl group, (c) a halo (C₁-C₆) alkyl group, (d) a (C₁-C₆) alkoxy group, (e) a halo (C₁-C₆) alkoxy group, (f) a (C₁-C₆) alkylthio group, (g) a halo (C₁-C₆) alkylthio group, (h) a (C₁-C₆) alkylsulfinyl group, (i) a halo (C₁-C₆)alkylsulfinyl group, (j) a (C₁-C₆)alkylsulfonyl group, (k) a halo (C₁-C₆) alkylsulfonyl group, (1) a nitro group, and (m) a trimethylsilyl group;
Y¹ and Y⁴ are identical or different, and represent
(d1) a hydrogen atom;
(d2) a halogen atom; or
(d3) a (C₁-C₆)alkyl group;
Y² and Y³ are identical or different, and represent
(e1) a hydrogen atom;
(e2) a halogen atom;
(e3) a (C₁-C₆)alkyl group;
(e5) a (C₁-C₆)alkylthio group;
(e6) a halo (C₁-C₆) alkyl group;
(e7) a halo (C₁-C₆) alkoxy group;
(e8) an aryloxy group;
(e10) an arylthio group; or
(e12) an arylcarbonyl group.

3. A canine filariasis control agent for an animal, comprising the benzimidazole compound or a salt thereof according to any one of claims 1 to 2 as an active ingredient.

4. A method of using a canine filariasis control agent for an animal, comprising administering an effective amount of the benzimidazole compound or a salt thereof according to any one of claims 1 to 2 to the animal.
